# EUROPEAN PATENT APPLICATION

(11) **EP 4 349 968 A1**
(43) Date of publication of application: **10.04.2024**
(21) Application number: 22816234.3
(22) Date of filing: 03.06.2022
(51) Int. Cl.: C12N 5/078, A61K 35/28, A61K 35/545, A61P 7/00, A61P 35/00, A61P 37/02, C12N 5/0783, C12N 5/10, C12N 15/12, C12N 15/13, C12N 15/62

(54) **CELL COMPOSITION, METHOD FOR PRODUCING CELL COMPOSITION, AND PHARMACEUTICAL COMPOSITION CONTAINING CELL COMPOSITION**

(30) Priority: 04.06.2021 JP 2021094716
(71) Applicant: KIRIN HOLDINGS KABUSHIKI KAISHA, Nakano-ku, Tokyo 164-0001 (JP)
(72) Inventor: TAKAYANAGI, Shinichiro, Tokyo 164-0001 (JP)
(74) Representative: Hoffmann Eitle
(86) International application number: PCT/JP2022/022661
(87) International publication number: WO 2022/255489

(57) **Abstract**

The purpose of the present invention is to provide a technique for concentrating blood cell-based cells having high proliferation ability and/or differentiation ability. The present invention relates to: a method for producing a cell composition, the method comprising a step for extracting cells which express G-protein-coupled receptor 56 (GPR56) from a cell aggregation containing immature blood cell-based cells; and a cell composition which comprises immature blood cell-based cells expressing GPR56 and in which the proportion of the immature blood cell-based cells expressing GPR56 is 50-100% of the total cells.

## Description

### TECHNICAL FIELD

The present invention relates to a method for obtaining blood cells having high proliferation ability and/or differentiation ability, and a cell composition containing a blood cell having high proliferation ability and/or differentiation ability.

### BACKGROUND ART

Pluripotent stem cells (PSC), such as embryonic stem cells (ES cell, ESC) and induced pluripotent stem cells (iPS cell, iPSC), retain the property of being able to be differentiated into various cells (pluripotency), and are expected to be used as a source for cell production in the field of regenerative medicine.

In recent years, various methods enable differentiation of PSCs into target cells. On the other hand, PSCs exhibit functional heterogeneity within clones due to genetic and epigenetic diversity. Therefore, in an actual differentiation process, not all PSCs are differentiated into target cells (Non Patent Literature 1). In addition, cells in the differentiation process are unstable (Non Patent Literature 2), resulting in even more heterogeneous cell aggregate after differentiation. Therefore, a technique for controlling differentiation of PSCs are being actively developed with the aim of producing uniform cell aggregates.

In a developmental process in a blood cell system, PSCs are differentiated into immature blood cells called multipotent hematopoietic stem cells (MHSC) such as hematopoietic stem cells (HSC) and hematopoietic progenitor cells (HPC), and then the immature blood cells are differentiated into various mature blood cells (Non Patent Literatures 3 and 4). Even in vitro, differentiation of PSCs into HSCs and HPCs has been reported (Non Patent Literature 5). HSCs and HPCs are generally characterized by expression of cell surface markers CD34 and/or CD43 (Non Patent Literatures 5 and 6).

On the other hand, in recent years, it has been reported that a G protein-coupled receptor 56 (hereinafter, GPR56) is expressed in HSC in a developmental period in vivo. GPR56 is known to be required for cluster formation of HSCs during an in vivo developmental process and to be important for generation of HSCs from vascular endothelial cells (Non Patent Literature 7). Further, it is known that although GPR56 is abundantly expressed only in early hematopoietic stem and progenitor cells during a hematopoietic step in mouse embryos or adult bone marrows, an expression level of GPR56 rapidly decreases as hematopoietic stem and progenitor cells are differentiated into mature blood lineages, and GPR56 expression does not affect maintenance of hematopoietic stem and progenitor cells or functions thereof (Non Patent Literature 8).

However, unlike conventional cell surface markers characterizing MHSCs, such as CD34 and CD43, GPR56 is not known to be involved in cell proliferation or differentiation in the differentiation process of MHSCs into mature blood cells.

### CITATION LIST

### NON PATENT LITERATURE

NPL1: Patrick Cahan & George Q, Daley, Origins and implications of pluripotent stem cell variability and heterogeneity; Nat. Rev. Mol. Cell Biol., 2013 Jun, 14(6), 357-368
NPL2: Taro Ichimura et al., Visualizing the appearance and disappearance of the attractor of differentiation using Raman spectral imaging; Sci. Rep., 2015, 5, 11358, 1-10
NPL3: Elisabetta Dejana et al., The molecular basis of endothelial cell plasticity; Nat. Commun., 2017 Feb 9, 8, 14361, 1-11
NPL4: Leo D Wang & Amy J Wagers, Dynamic niches in the origination and differentiation of haematopoietic stem cells; Nat. Rev. Mol. Cell Biol., 2011 Sep 02, 12(10), 643-655
NPL5: Christopher M. Sturgeon et al., Wnt Signaling Controls the Specification of Definitive and Primitive Hematopoiesis From Human Pluripotent Stem Cells; Nat. Biotechnol., 2014 Jun, 32(6), 554-561
NPL6: Maxim A. Vodyanik et al, Leukosialin (CD43) defines hematopoietic progenitors in human embryonic stem cell differentiation cultures; Blood, 2006 Sep 15, 108(6), 2095-2105
NPL7: Parham Solaimani Kartalaei et al., Whole-transcriptome analysis of endothelial to hematopoietic stem cell transition reveals a requirement for Gpr56 in HSC generation; J. Exp. Med., 2015 Jan 12, 212(1), 93-106
NPL8: Tata Nageswara Rao et al., High-level Gpr56 expression is dispensable for the maintenance and function of hematopoietic stem and progenitor cells in mice; Stem Cell Res., 2015 May, 14(3), 307-322

### SUMMARY OF INVENTION

### TECHNICAL PROBLEM

As described above, in the actual PSC differentiation process, not all PSCs are differentiated into target cells. In addition, cells in the differentiation process are unstable, resulting in even more heterogeneous cell aggregate after differentiation. Therefore, although a technique for controlling differentiation of PSCs are being actively developed with the aim of producing uniform cell aggregates, no industrially applicable technique has yet been found.

The present inventors have found for the first time that a cell aggregate conventionally characterized as MHSCs by expression of CD34 and/or CD43 contains cells that are not proliferated sufficiently or not differentiated into target cells in subsequent culture, and therefore, a cell composition containing blood cells obtained by differentiating conventional MHSCs cannot be stably produced in a large amount.

Therefore, an object of the present invention is to provide a technique for concentrating blood cells having high proliferation ability and/or differentiation ability.

### SOLUTION TO PROBLEM

As a result of intensive studies to solve the above problems, the present inventor has found that GPR56 is involved in proliferation ability and/or differentiation ability of cells in a differentiation process of immature blood cells into mature blood cells, and that blood cells having high proliferation ability and/or differentiation ability can be concentrated by extracting cells expressing GPR56 from a cell aggregate containing immature blood cells, thereby completing the present invention.

That is, the present invention provides the following inventions.
[1] A method for producing a cell composition, the method including: a step of extracting cells expressing GPR56 from a cell aggregate containing immature blood cells.
[2] The production method according to [1], in which the cell expressing GPR56 is a human cell.
[3] The production method according to [1] or [2], in which the cell expressing GPR56 is a cell expressing CD34 along with GPR56.
[4] The production method according to any one of [1] to [3], in which the cell expressing GPR56 expresses at least one of CD43 and CD45 along with GPR56.
[5] The production method according to any one of [1] to [4], in which the cell aggregate is a cell aggregate derived from pluripotent stem cells.
[6] The production method according to [5], in which the cell aggregate is a cell aggregate obtained by inducing differentiation of the pluripotent stem cells into mesoderm and then culturing the mesoderm in a medium containing a stem cell factor (hereinafter, "SCF"), a thrombopoietin recombinant protein (hereinafter, "TPO"), and an Fms-related tyrosinase kinase 3 ligand (hereinafter, "Flt3-L").
[7] The production method according to [5], in which the cell aggregate derived from the pluripotent stem cells is a cell aggregate obtained by inducing differentiation of the pluripotent stem cells into hematopoietic stem cells or hematopoietic progenitor cells.
[8] The production method according to any one of [1] to [7], in which the cell aggregate is a cell aggregate containing at least one of hematopoietic stem cells and hematopoietic progenitor cells.
[9] The production method according to any one of [1] to [8], in which the cell aggregate is a cell aggregate containing cells expressing at least one selected from CD34, CD43, and CD45.
[10] The production method according to any one of [1] to [9], further including: a step of inducing differentiation of the cells expressing GPR56 into mature blood cells.
[11] The production method according to [10], in which the mature blood cell is a lymphoid cell.
[12] The production method according to [11], in which the lymphoid cell is a T cell or a natural killer cell.
[13] The production method according to [11], in which the lymphoid cell is a cell expressing a chimeric antigen receptor.
[14] The production method according to [10], in which the mature blood cell is a myeloid cell.
[15] The production method according to any one of [10] to [14], further including: a step of culturing and proliferating cells before or after inducing differentiation into the mature blood cells.
[16] A cell composition containing: an immature blood cell expressing GPR56, in which a proportion of the immature blood cell expressing GPR56 is 50% to 100% of total cells.
[17] The cell composition according to [16], in which the immature blood cell expressing GPR56 is a human cell.
[18] The cell composition according to [16] or [17], in which the immature blood cell expressing GPR56 is a hematopoietic stem cell or a hematopoietic progenitor cell.
[19] The cell composition according to any one of [16] to [18], in which the immature blood cell expressing GPR56 is a cell expressing CD34 along with GPR56.
[20] The cell composition according to any one of [16] to [19], in which the immature blood cell expressing GPR56 is a cell expressing at least one of CD43 and CD45 along with GPR56.
[21] The cell composition according to any one of [16] to [20], in which the immature blood cell expressing GPR56 is a cell derived from a pluripotent stem cell.
[22] The cell composition according to [21], in which the immature blood cell expressing GPR56 is a cell obtained by inducing differentiation of the pluripotent stem cell into mesoderm and then culturing the mesoderm in a medium containing SCF, TPO, and Flt3-L.
[23] The cell composition according to [21] or [22], in which the immature blood cell expressing GPR56 is a cell obtained by inducing differentiation of the pluripotent stem cell into a hematopoietic stem cell or a hematopoietic progenitor cell.
[24] A pharmaceutical composition containing: a cell composition produced by the production method according to any one of [1] to [15].
[25] The pharmaceutical composition according to [24], in which the pharmaceutical composition is a therapeutic agent or blood preparation for at least one selected from cancer, an immune disease, and a blood disease.
[26] A pharmaceutical composition containing: the cell composition according to any one of [16] to [23].
[27] The pharmaceutical composition according to [26], in which the pharmaceutical composition is a therapeutic agent or blood preparation for at least one selected from cancer, an immune disease, and a blood disease.
   [1'] A method for producing a cell composition, the method including: a step of extracting cells expressing a G protein-coupled receptor 56 (hereinafter, referred to as GPR56) from a cell aggregate containing immature blood cells.
   [2'] The production method according to [1'], in which the cell expressing GPR56 is a human cell.
   [3'] The production method according to [1'], in which the cell expressing GPR56 is a cell expressing CD34 along with GPR56.
   [4'] The production method according to [1'], in which the cell expressing GPR56 expresses at least one of CD43 and CD45 along with GPR56.
   [5'] The production method according to [1'], in which the cell aggregate is a cell aggregate derived from pluripotent stem cells.
   [6'] The production method according to [5'], in which the cell aggregate is a cell aggregate obtained by inducing differentiation of the pluripotent stem cells into mesoderm and then culturing the mesoderm in a medium containing a stem cell factor (SCF), a thrombopoietin recombinant protein (TPO), and an Fms-related tyrosinase kinase 3 ligand (Flt3 -L).
   [7'] The production method according to [5'], in which the cell aggregate derived from the pluripotent stem cells is a cell aggregate obtained by inducing differentiation of the pluripotent stem cells into hematopoietic stem cells or hematopoietic progenitor cells.
   [8'] The production method according to [1'], in which the cell aggregate is a cell aggregate containing at least one of hematopoietic stem cells and hematopoietic progenitor cells.
   [9'] The production method according to [1'], in which the cell aggregate is a cell aggregate containing cells expressing at least one selected from CD34, CD43, and CD45.
   [10'] The production method according to [1'], further including: a step of inducing differentiation of the cells expressing GPR56 into mature blood cells.
   [11'] The production method according to [10'], in which the mature blood cell is a lymphoid cell.
   [12'] The production method according to [11'], in which the lymphoid cell is a T cell or a natural killer cell.
   [13'] The production method according to [11'], in which the lymphoid cell is a cell expressing a chimeric antigen receptor.
   [14'] The production method according to [10'], in which the mature blood cell is a myeloid cell.
   [15'] The production method according to [10'], further including: a step of culturing and proliferating cells before or after inducing differentiation into the mature blood cells.
   [16'] A cell composition containing: an immature blood cell expressing GPR56, in which a proportion of the immature blood cell expressing GPR56 is 50% to 100% of total cells.
   [17'] The cell composition according to [16'], in which the immature blood cell expressing GPR56 is a human cell.
   [18'] The cell composition according to [16'], in which the immature blood cell expressing GPR56 is a hematopoietic stem cell or a hematopoietic progenitor cell.
   [19'] The cell composition according to [16'], in which the immature blood cell expressing GPR56 is a cell expressing CD34 along with GPR56.
   [20'] The cell composition according to [16'], in which the immature blood cell expressing GPR56 is a cell expressing at least one of CD43 and CD45 along with GPR56.
   [21'] The cell composition according to [16'], in which the immature blood cell expressing GPR56 is a cell derived from a pluripotent stem cell.
   [22'] The cell composition according to [21'], in which the immature blood cell expressing GPR56 is a cell obtained by inducing differentiation of the pluripotent stem cell into mesoderm and then culturing the mesoderm in a medium containing SCF, TPO, and Flt3-L.
   [23'] The cell composition according to [21'], in which the immature blood cell expressing GPR56 is a cell obtained by inducing differentiation of the pluripotent stem cell into a hematopoietic stem cell or a hematopoietic progenitor cell.
   [24'] A pharmaceutical composition containing: a cell composition produced by the production method according to any one of [1'] to [15'].
   [25'] The pharmaceutical composition according to [24'], in which the pharmaceutical composition is a therapeutic agent or blood preparation for at least one selected from cancer, an immune disease, and a blood disease.
   [26'] A pharmaceutical composition containing: the cell composition according to any one of [16'] to [23'].
   [27'] The pharmaceutical composition according to [26'], in which the pharmaceutical composition is a therapeutic agent or blood preparation for at least one selected from cancer, an immune disease, and a blood disease.

### ADVANTAGEOUS EFFECTS OF INVENTION

According to the method for producing a cell composition of the present invention, by including a step of extracting cells expressing GPR56 from a cell aggregate containing immature blood cells, blood cells having high proliferation ability and/or high differentiation ability can be concentrated, and a cell composition containing a blood cell can be stably produced in a large amount.

According to the cell composition of the present invention, since the cell composition contains an immature blood cell expressing GPR56, and a proportion of the immature blood cell expressing GPR56 is 50% to 100% of total cells, excellent proliferation ability and/or differentiation ability can be exhibited and a cell composition containing a blood cell can be stably and efficiently produced.

### BRIEF DESCRIPTION OF DRAWINGS

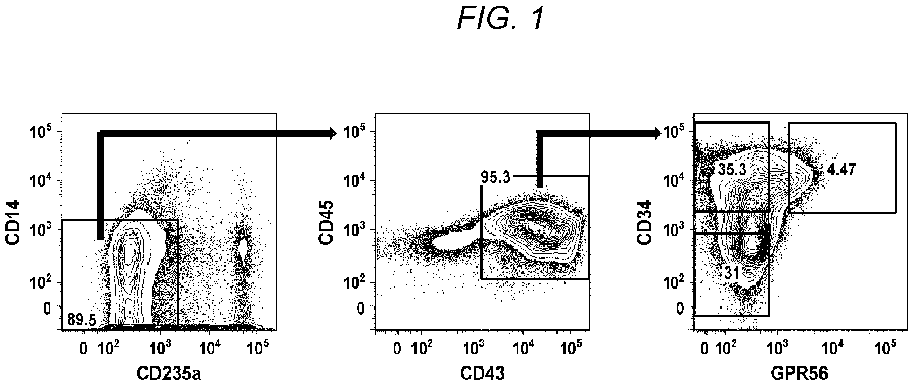

[FIG. 1] FIG. 1 is a diagram showing results of analyzing cell surface markers of an embryoid body (EB) obtained by inducing differentiation of a T cell-derived iPS cell line (TkT3V1-7) into hematopoietic stem cells, in which as indicated by arrows, cell aggregates within square frames in left, middle, and right figures are gated and subjected to analysis using the following cell marker, a vertical axis and a horizontal axis in each figure indicate an expression level of each cell surface marker, and a numerical value in the square frame in each figure indicates a proportion of the number of cells in the region to the total number of cells.

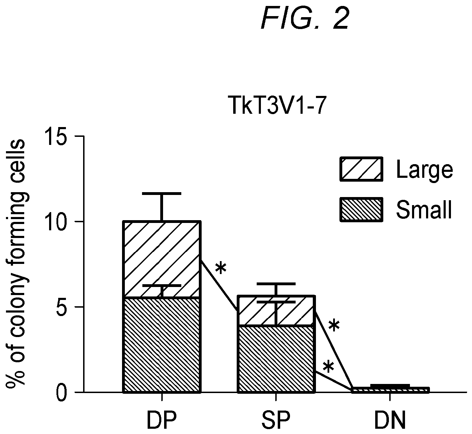

[FIG. 2] FIG. 2 is a diagram showing results of evaluating colony forming ability after gating, from a PI(-)CD14(-)CD235a(-)CD43(+)CD45(low+) cell aggregate among EB cells obtained from the T cell-derived iPS cell line (TkT3V1-7), a CD34(-)GPR56(-) double negative (denoted as DN in the figure) cell aggregate, a CD34(+)GPR56(-) single positive (denoted as SP in the figure) cell aggregate, and a CD34(+)GPR56(+) double positive (denoted as DP in the figure) cell aggregate, separately, and inducing differentiation into myeloid cells, in which a vertical axis indicates a proportion of the number of cells forming a colony to the total number of cells, and * indicates that there is a statistically significant difference between shown data.

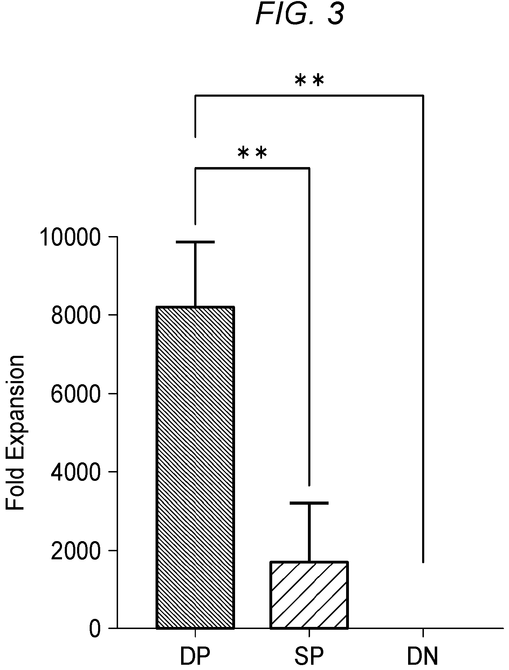

[FIG. 3] FIG. 3 is a diagram showing results of counting the number of living cells in each cell aggregate after gating, from aPI(-)CD14(-)CD235a(-)CD43(+)CD45(low+) cell aggregate among EB cells obtained from the T cell-derived iPS cell line (TkT3V1-7), a CD34(-)GPR56(-) double negative (denoted as DN in the figure) cell aggregate, a CD34(+)GPR56(-) single positive (denoted as SP in the figure) cell aggregate, and a CD34(+)GPR56(+) double positive (denoted as DP in the figure) cell aggregate, separately, and inducing differentiation into T cells, in which a vertical axis indicates a value obtained by dividing the final number of living cells after culture by the number of initially seeded cells, and ** indicates that there is a statistically significant difference between shown data.

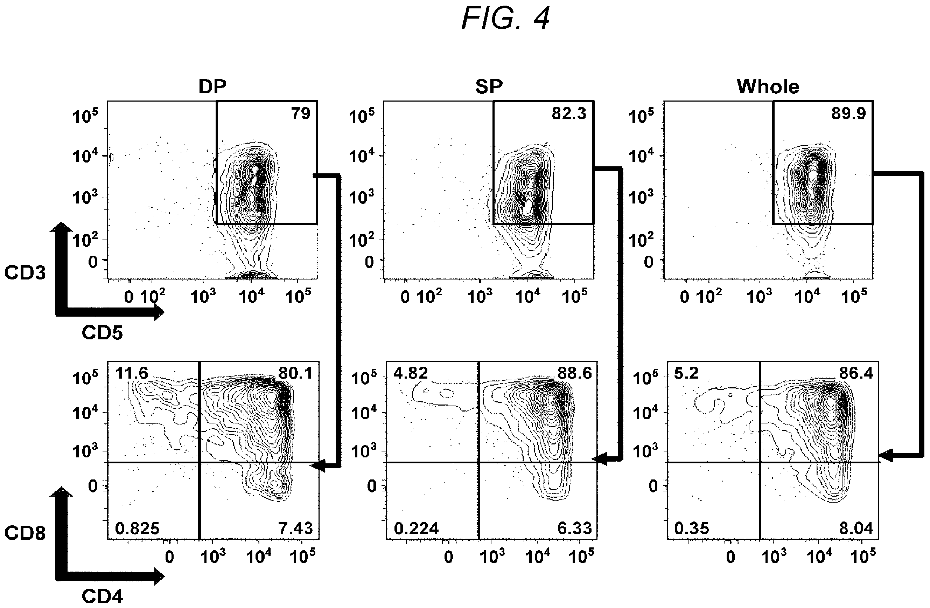

[FIG. 4] FIG. 4 is a diagram showing results of analyzing cell surface markers of each cell aggregate after gating, from aPI(-)CD14(-)CD235a(-)CD43(+)CD45(low+) cell aggregate (denoted as Whole in the figure) among EB cells obtained from the T cell-derived iPS cell line (TkT3V1-7), a CD34(+)GPR56(-) single positive (indicated as SP in the figure) cell aggregate and a CD34(+)GPR56(+) double positive (denoted as DP in the figure) cell aggregate, and inducing differentiation into T cells, in which as indicated by arrows, among the cell aggregates shown in upper left, middle, and right figures, cell aggregates within upper right square frames are gated, expanded to lower left, middle, and right figures, respectively, and subjected to analysis using the following cell surface marker, a vertical axis and a horizontal axis in each figure indicate an expression level of each cell surface marker, and a numerical value in the square frame in each figure indicates a proportion of the number of cells in the region to the total number of cells.

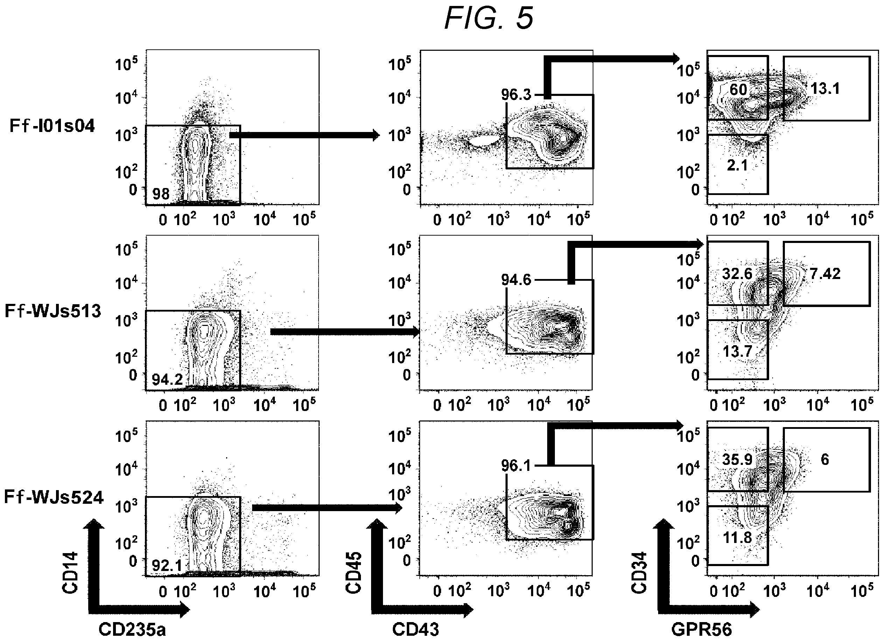

[FIG. 5] FIG. 5 shows results of analyzing cell surface markers of EBs obtained by inducing differentiation of Ff-I01s04, Ff-WJs513, and Ff-WJs524, which are Non-T-iPSCs, into hematopoietic stem cells, in which as indicated by arrows, in each cell line, cell aggregates within square frames in left, middle, and right figures are gated and subjected to analysis using the following cell marker, a vertical axis and a horizontal axis in each figure indicate an expression level of each cell surface marker, and a numerical value in the square frame in each figure indicates a proportion of the number of cells in the region to the total number of cells.

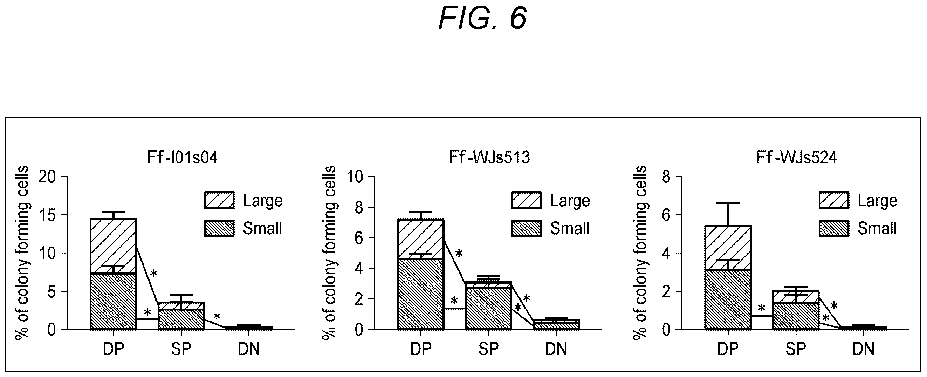

[FIG. 6] FIG. 6 is a diagram showing results of evaluating colony forming ability after gating, from a PI(-)CD14(-)CD235a(-)CD43(+)CD45(low+) cell aggregate among EB cells obtained from Ff-I01s04, Ff-WJs513, and Ff-WJs524, which are Non-T-iPSCs, a CD34(-)GPR56(-) double negative (denoted as DN in the figure) cell aggregate, a CD34(+)GPR56(-) single positive (denoted as SP in the figure) cell aggregate, and a CD34(+)GPR56(+) double positive (denoted as DP in the figure) cell aggregate, separately, and inducing differentiation into myeloid cells, in which a vertical axis in each figure indicates a proportion of the number of cells forming a colony to the total number of cells, and * indicates that there is a statistically significant difference between shown data.

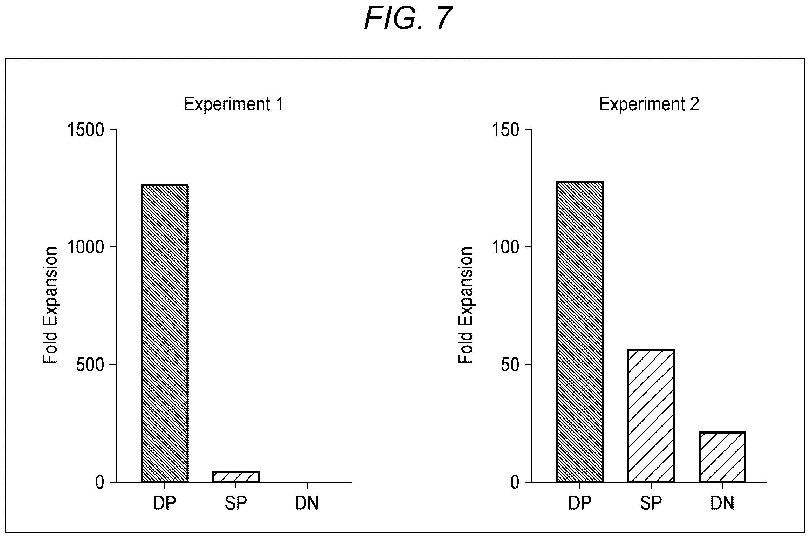

[FIG. 7] FIG. 7 is a diagram showing results of counting the number of living cells in each cell aggregate after gating, from aPI(-)CD14(-)CD235a(-)CD43(+)CD45(low+) cell aggregate among EB cells obtained from Ff-I01s04, which is Non-T-iPSC, a CD34(-)GPR56(-) double negative (denoted as DN in the figure) cell aggregate, a CD34(+)GPR56(-) single positive (denoted as SP in the figure) cell aggregate, and a CD34(+)GPR56(+) double positive (denoted as DP in the figure) cell aggregate, separately, and inducing differentiation into NK cells, in which "Experiment 1" and "Experiment 2" shown in the figure each indicate results of two independent experiments, and a vertical axis in each figure indicates a value obtained by dividing the final number of living cells after culture by the number of initially seeded cells.

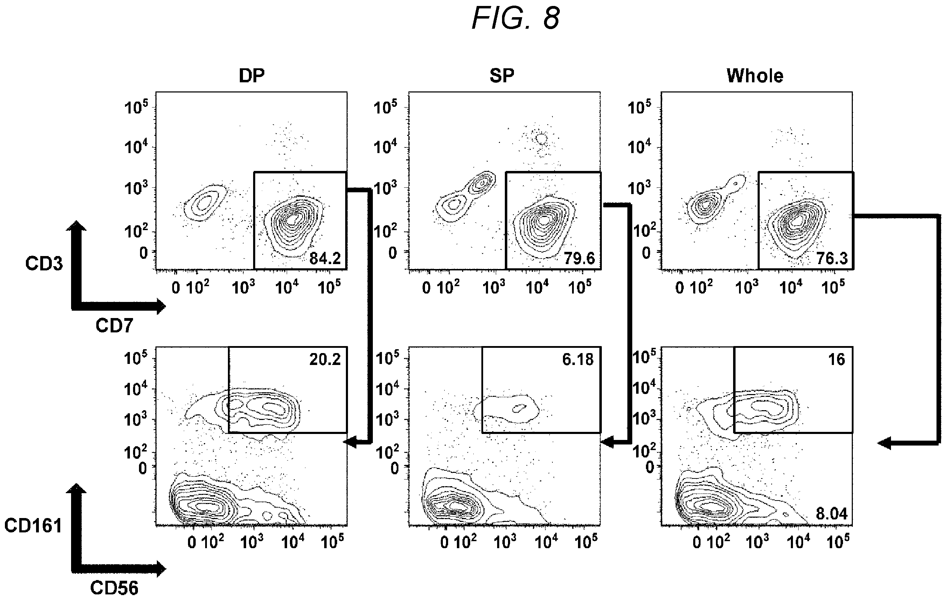

[FIG. 8] FIG. 8 is a diagram showing results of analyzing cell surface markers of each cell aggregate after gating, from aPI(-)CD14(-)CD235a(-)CD43(+)CD45(low+) cell aggregate (denoted as Whole in the figure) among EB cells obtained from a Ff-I01s04 line, which is Non-T-iPSC, a CD34(+)GPR56(-)single positive (indicated as SP in the figure) cell aggregate and a CD34(+)GPR56(+) double positive (denoted as DP in the figure) cell aggregate, and inducing differentiation into NK cells, in which among the cell aggregates shown in upper left, middle, and right figures, cell aggregates in upper right square frames are gated, expanded to lower left, middle, and right figures, respectively, and subjected to analysis using the following cell surface marker, a vertical axis and a horizontal axis in each figure indicate an expression level of each cell surface marker, and a numerical value in the square frame in each figure indicates a proportion of the number of cells in the region to the total number of cells.

### DESCRIPTION OF EMBODIMENTS

The present invention will be described in detail below.

### <Method for Producing Cell Composition>

The present invention relates to a method for producing a cell composition, the method including a step of extracting cells expressing GPR56 from a cell aggregate containing immature blood cells.

In the present invention, the "blood cell" means MHSC and various cells obtained by differentiating MHSC, and examples thereof include all blood cells including immature blood cells and mature blood cells. Examples of the blood cell include HSC, HPC, a common lymphoid progenitor cell, a common myeloid progenitor cell, B cell-NK cell progenitor cell, an erythroid progenitor cell, a granulocyte-monocyte progenitor cell, a natural killer (NK) cell, a B cell, a T cell, a NK/T cell, a monocyte, a macrophage, a dendritic cell, a neutrophil, an eosinophil, a basophil, a mast cell, a megakaryocyte, and a red blood cell. When the blood cell is a NK cell or a T cell, the NK cell or T cell may be a cell expressing a chimeric antigen receptor (CAR) to be described later.

In the present invention, the "immature blood cell" means MHSC and various progenitor cells obtained by differentiating MHSC among the blood cells. Examples of the immature blood cell include HSC, HPC, a common lymphoid progenitor cell, a B cell-NK cell progenitor cell, a common myeloid progenitor cell, an erythroid progenitor cell, and a granulocyte-monocyte progenitor cell. HSC or HPC is preferred.

HSC is a stem cell having self-replicating ability and ability to be differentiated into all blood cells including HPC. HPC is a blood progenitor cell obtained by differentiating HSC, and has an ability to be differentiated into all blood cells excluding HSC. Examples of a positive marker for identifying HSC and HPC include CD34, CD43, and CD45.

The positive marker is also referred to as a cell surface marker, and is a molecule that can be expressed on the surface of a target cell. By detecting expression of the positive marker, target cells can be detected and separated. For detection of a positive marker, a reagent containing an antibody specific to a target positive marker labeled with various fluorescent dyes such as known green fluorescent protein (GFP), fluorescein isothiocyanate (FITC), a phycoerthrin (PE), or an allophycocyanin (APC) can be used.

Examples of a method used for detecting and separating target cells include a method using flow cytometry such as BD FACSympone flow cytometer (BD Bioscience), or mass cytometry such as Helios mass cytometer (Fluidigm), and a magnetic cell separation method such as a method using a CD34 microbead kit (Miltenyi Biotec) or a method described in Miltenyi S, et al.; Cytometry, 1990, 11, 231. A cell expressing a target positive marker can be selected, extracted, and/or concentrated by using the method used for detecting and separating target cells.

In the present invention, the term "express" means that a target positive marker can be detected by the above-described method used for detecting and separating target cells. On the other hand, the term "not express" means that a target positive marker cannot be detected within a detection sensitivity range of the method used for detecting and separating target cells.

In the present invention, the term "cell aggregate" means an aggregate of a plurality of cells. In the present invention, the term "cell composition" means a composition containing cells or a cell aggregate. The cell aggregate may be composed of only a single type of cell or a plurality of types of cells. The cell composition may contain components other than cells, such as a culture medium and a preservation solution.

That is, the term "cell aggregate containing immature blood cells" is an aggregate of a plurality of cells containing the above-described immature blood cells. Examples of the cell aggregate containing immature blood cells include an aggregate of a plurality of cells containing HSC, HPC, common lymphoid progenitor cells, B cell-NK cell progenitor cells, common myeloid progenitor cells, erythroid progenitor cells or granulocyte-monocyte progenitor cells, and the like. The cell aggregate containing immature blood cells is preferably an aggregate of a plurality of cells containing at least one of HSC and HPC. The cell aggregate containing immature blood cells may be, for example, a cell aggregate containing cells expressing at least one selected from CD34, CD43 and CD45 as a positive marker.

The production method of the present invention may include, before the step of extracting cells expressing GPR56 from a cell aggregate containing immature blood cells, a step of providing or preparing a cell aggregate containing immature blood cells.

Examples of a derivation and a preparation method of the cell aggregate containing immature blood cells include, but are not particularly limited to, a method of inducing differentiation of pluripotent stem cells in vivo or in vitro according to a known method described in Suzuki N, et al.; Mol. Ther., 2013, 7, 1424, Takayama M, et al.; J. Exp. Med., 2010, 207, 2817 or the like, and a method of collecting from biological tissue using a known method.

When the cell aggregate containing immature blood cells is derived from pluripotent stem cells, the cell aggregate is preferably a cell aggregate obtained by inducing differentiation of pluripotent stem cells into MHSCs such as HSCs or HPCs, or a cell aggregate obtained by inducing differentiation of pluripotent stem cells into mesoderm and then culturing the mesoderm in a medium containing a stem cell factor (SCF), a thrombopoietin recombinant protein (TPO), and a Fms-related tyrosine kinase 3 ligand (Flt3-L). The cell aggregate is more preferably a cell aggregate obtained by inducing differentiation of pluripotent stem cells into HSCs or HPCs.

In the present invention, the term "cell aggregate obtained by inducing differentiation" means a cell aggregate subjected to processing for the purpose of inducing differentiation. In the present invention, the term "cell obtained by inducing differentiation" means a cell subjected to processing for the purpose of inducing differentiation.

Examples of a method for inducing differentiation of pluripotent stem cells into mesoderm and then culturing the mesoderm in a medium in vitro to induce differentiation into MHSCs include a known method of inducing differentiation of iPSCs into mesoderm and then culturing the mesoderm in a medium in vitro to induce differentiation into MHSCs. Specific examples of the method include a method using an embryoid body (EB) formation method described in WO2020/138371.

A concentration of the SCF in the medium in the differentiation induction step is preferably 1 ng/ml to 200 ng/ml, and particularly preferably about 50 ng/ml. A concentration of the TPO in the medium in the differentiation induction step is preferably 1 ng/ml to 100 ng/ml, and particularly preferably about 30 ng/ml. A concentration of the Flt3-L in the medium in the differentiation induction step is preferably 1 ng/ml to 100 ng/ml, and particularly preferably about 10 ng/ml. Here, the term "about" means including a range of ±10%.

The pluripotent stem cell is a cell retaining a property of being able to be differentiated into various cells, and examples thereof include ESC, iPSC, an embryonic tumor cell, and an embryonic germ stem cell. iPSC is preferred. Examples of a derivation and an acquisition method of the pluripotent stem cells include, but are not particularly limited to, a method of preparing pluripotent stem cells from somatic cells or the like by a known method, and a method of collecting pluripotent stem cells from biological tissue by a known method.

Examples of an animal from which a pluripotent stem cell, a somatic cell, or biological tissue is derived include, but are not particularly limited to, mammals such as a mouse, a rat, a hamster, a guinea pig, a dog, a monkey, an orangutan, a chimpanzee, and a human. A human is preferred. The biological tissue is not particularly limited as long as it contains immature blood cells, and examples thereof include peripheral blood, a lymph node, bone marrow, thymus, pancreas, and umbilical cord blood.

When the pluripotent stem cell is derived from a somatic cell, the somatic cell is not limited, and examples thereof include a T cell, a peripheral blood-derived cell, and an umbilical cord blood-derived cell. When the somatic cell is a T cell, the T cell may be a cell expressing a chimeric antigen receptor to be described later.

When the pluripotent stem cell is iPSC, the iPSC may be produced from a somatic cell by a known production method to be described later, may be obtained and used from public banks that are already established and stocked, such as the JCRB cell bank of National Institutes of Biomedical Innovation, Health and Nutrition, or the American Type Culture Collection, or may be obtained and used as a commercially available reagent or the like from Takara Bio Inc., or the like.

The iPSC can be produced by introducing an initialization factor into any somatic cell. Examples of the initialization factor include a gene or a gene product such as Oct3/4, Sox2, Sox1, Sox3, Sox15, Sox17, Klf4, Klf2, c-Myc, N-Myc, L-Myc, Nanog, Lin28, Fbx15, Eras, ECAT15-2, Tcl1, beta-catenin, Lin28b, Sall1, Sall4, Esrrb, Nr5a2, Tbx3, and Glis1. These initialization factors may be used alone, or a plurality of factors may be used in combination.

Examples of the iPSC production method include methods described in WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al.; Nat. Biotechnol., 2008, 26, 795, Shi Y, et al.; Cell Stem Cell, 2008, 2, 525, Eminli S, et al.; Stem Cells, 2008, 26, 2467, Huangfu D, et al.; Nat. Biotechnol., 2008, 26, 1269, Shi Y, et al.; Cell Stem Cell, 2008, 3, 568, Zhao Y, et al.; Cell Stem Cell, 2008, 3, 475, Marson A.; Cell Stem Cell, 2008, 3, 132, Feng B, et al.; Nat. Cell Biol., 2009, 11, 197, R.L. Judson et al.; Nat. Biotechnol., 2009, 27, 459, Lyssiotis CA, et al.; Proc. Natl. Acad. Sci. USA; 2009, 106, 8912, Kim J. B., et al.; Nature, 2009, 461, 649, Ichida J. K., et al.; Cell Stem Cell., 2009, 5, 491, Heng J. C., et al.; Cell Stem Cell, 2010, 6, 167, Han J, et al.; Nature, 2010, 463, 1096, Mali P, et al.; Stem Cells, 2010, 28, 713, and Maekawa M, et al.; Nature, 2011, 474, 225. These production methods may be used alone, or a plurality of production methods may be used in combination.

In the present invention, the term "cell expressing GPR56" is a cell expressing GPR56 as a positive marker.

GPR56 is a gene product referred to as ADHESION G PROTEIN-COUPLED RECEPTOR G1 (ADGRG1), 7-TRANSMEMBRANE PROTEIN WITH NO EGF-LIKE N-TERMINAL DOMAINS 1 (TM7XN1), BILATERAL FRONTOPARIETAL POLYMICROGYRIA (BFPP), POLYMICROGYRIA, BILATERAL, PERISYLUIAN, AUTOSOMAL, RECESSIVE (BPPR), or TM7LN4.

Examples of the derivation of GPR56 in the present invention include, but are not particularly limited to, a human, a monkey, and a mouse. A human is preferred. GPR56 expressed in a cell may be a full-length sequence or a partial sequence as a protein. Examples of GPR56 as a protein include an adhesion G-protein coupled receptor G1 isoform b precursor (National Center for Biotechnology Information, NCBI ACCESSION No. NP_001139242.1), and examples of a nucleic acid encoding the protein include ADGRG1, transcript variant 5, mRNA (National Center for Biotechnology Information, NCBI ACCESSION No. NM_001145770.3).

Examples of the cell expressing GPR56 include, but are not particularly limited to, a blood cell. Specific examples of the cell expressing GPR56 include HSC and HPC, and further include a cell expressing a positive marker for identifying HSC and HPC along with GPR56. Specific examples thereof include a cell expressing CD34 along with GPR56, a cell expressing CD45 along with GPR56, a cell expressing CD43 along with GPR56, a cell expressing CD34, and CD43 and/or CD45 along with GPR56, and a cell expressing CD43, and CD45 along with GPR56.

The cell expressing GPR56 in the present invention has high proliferation ability and/or differentiation ability, and the ability thereof can be confirmed by proliferating or differentiating cells by a known proliferation method or differentiation method and comparing the cells with cells not expressing GPR56.

As the proliferation method of cells expressing GPR56, a known method of proliferating cells of a human or another animal can be used, and examples thereof include a proliferation method using, for example, an anti-CD3 antibody when the cells are CD4⁺ T cells, and a proliferation method using, for example, a medium containing CF and/or TPO when the cells are HSC or HPC.

When the cells expressing GPR56 of the present invention are immature cells such as various progenitor cells, the cells may be further differentiated into mature cells.

Examples of the method for inducing differentiation of the cells expressing GPR56 include a method described in WO2016/076415 or WO2017/221975 when the cells are MHSCs and are induced to be differentiated into CD8-positive cells. When the cells are HSC or HPC and are induced to be differentiated into relatively mature cells such as red blood cells or granulocyte cells, for example, a method using a medium containing an erythropoietin (EPO) and/or a granulocyte colony stimulating factor (G-CSF) used in a colony formation test or the like, or a method described in Notta F, et al.; Science, 2016, 351, 139 may be used.

The mature blood cell refers to a cell that is more differentiated than the immature blood cell. Examples of the mature blood cell include lymphoid cells such as a NK cell, a helper T cell, a cytotoxic T cell, and a B cell, and myeloid cells such as a macrophage, a dendritic cell, a neutrophil, a basophil, an eosinophil, a mast cells, a megakaryocyte, and a red blood cell. When the mature blood cell is a NK cell or a T cell, the NK cell or the T cell may be a cell expressing a chimeric antigen receptor (CAR).

The cell expressing CAR is genetically modified so as to express CAR and has enhanced specificity to cancer cells. Examples of the cell expressing CAR include a T cell expressing CAR (referred to as a CAR-T cell) and a NK cell expressing CAR (referred to as a CAR-NK cell or CAR-ILC). The CAR-T cell is preferably a cytotoxic T cell expressing CAR.

CAR is a fusion protein containing an extracellular domain binding to an antigen and an intracellular domain derived from a polypeptide different from the extracellular domain. Examples of CAR include a fusion protein obtained by binding an antigen recognition site (for example, a light chain (L chain) of a variable region and a heavy chain (H chain) of a variable region) of an antibody for a specific antigen to an intracellular domain of a T cell receptor such as CD3, or an intracellular domain of a T cell receptor such as CD3 and an intracellular domain of a costimulatory molecule such as CD28 or 4-1BB (for example, described in JP2015-509716A).

The antigen recognition site of CAR can be selected according to a target antigen, and NK cells or T cells more specific to the target antigen can be produced. For example, when CD19 is used as the antigen, an antigen recognition site of an anti-CD19 antibody is cloned, and the antigen recognition site is bound to an intracellular domain of a CD3 molecule to prepare CAR (for example, described in Claudia M, et al.; Cancer. Res., 2006, 66, 10995). The type, number, or the like of the costimulatory molecules to be bound can be appropriately selected to control the strength or duration of activation (for example, described in Michael C, et al.; Mol. Ther., 2009, 17, 1453).

CAR genes may be introduced into cells at any step in the production of the cell composition. Specifically, in production of a CAR-T cell composition, after introducing CARs into somatic cells from which iPSCs are derived, the somatic cells into which CARs are introduced may be induced to be differentiated into T cells via iPSCs, or after introducing CARs into iPSCs, the iPSCs may be induced to be differentiated into T cells, or after inducing differentiation of iPSCs into T cells, CARs may be introduced into the T cells.

The cell aggregate containing immature blood cells, the cell expressing GPR56, or the mature blood cell of the present invention can be obtained by each of the above-described methods, and can also be obtained by the following methods.

### «Step of Extracting Cells Expressing GPR56 from Cell Aggregate Containing Immature Blood Cells»

In the present invention, as an aspect of the step of extracting cells expressing GPR56 from a cell aggregate containing immature blood cells, a step of selectively extracting cells expressing GPR56 from a cell aggregate is included. As an aspect of the step of selectively extracting cells expressing GPR56, a step of detecting cells expressing GPR56 and separating the detected cells from a cell aggregate is included.

In the step of extracting cells expressing GPR56 from a cell aggregate containing immature blood cells, the above-described method using a substance specifically binding to GPR56 and flow cytometry or mass cytometry including a cell analyzer, a cell sorter, or the like, or the above-described magnetic cell separation method can be used.

For example, as the substance specifically binding to GPR56, a substance with a detectable label such as a green fluorescent protein (GFP) added to a known anti-GPR56 antibody such as a PE anti-human GPR56 antibody (BioLegend) can be used.

When using a substance with a label added, the substance is brought into contact with a cell aggregate containing cells expressing GPR56, and then the label is detected by a cell sorter, and the cells with the labeled substance bound can be separated.

For example, when using a substance without a label added, such as an anti-GPR56 antibody, separation can be performed in the same manner as described above by bringing the substance into contact with a cell aggregate containing cells expressing GPR56 and then bringing another substance with a detectable label for directly or indirectly recognizing the substance added into contact with the cell aggregate containing cells expressing GPR56.

Specifically, when the substance without a label added is an antibody, a fluorescent dye such as FITC, PE, or APC, a metal isotope such as 169 Tm or 164 Dm, or a magnetic bead such as Dynabeads (Thermo Fisher) or Magnetic Cell Sorting (MACS) is directly or indirectly bound to the antibody, whereby GPR56 on the cell surface can be labeled and/or cells expressing GPR56 can be separated.

Examples of a method for directly binding a fluorescent dye, a metal isotope, or a magnetic bead to the antibody without a label added include a method using an antibody or the like which is against the antibody and to which a fluorescent dye, a metal isotope, or a magnetic bead is bound (described in WO2009/026168). Examples of the method for indirectly binding a fluorescent dye, a metal isotope, or a magnetic bead to the antibody without a label added include a covalent bond with an amino group (described in Baumgart S, et al.; Eur. J. Immunol., 2017, 47, 1377).

### <<Step of Inducing Differentiation of Cells Expressing GPR56 into Mature Blood Cells>>

The production method of the present invention can further include a step of inducing differentiation of cells expressing GPR56 into mature blood cells. As the step of inducing differentiation of cells expressing GPR56 into mature blood cells, a known method of inducing differentiation of MHSCs into various blood cells can be used. Examples of the method for producing CD4/CD8 double-positive T cells include a method described in WO2017/221975. Examples of the method for producing cells such as red blood cells and megakaryocytes include a method described in Notta F, et al.; Science, 2016, 351, 139.

Examples of the method for inducing differentiation of MHSCs into T cells include the same or similar methods as those described above, and specific examples thereof include a method described in Ross N, et al.; Blood, 2005, 105(4), 1431.

The production method of the present invention may further include a step of culturing and proliferating cells before or after the step of inducing differentiation of cells expressing GPR56 into mature blood cells. For example, in the case of producing a CAR-T cell preparation, after extracting cells expressing GPR56, the cells can be proliferated for about 3 weeks under conditions of culture using Delta-like (DLL) 4 by a method described in Iriguchi S, et al.; Nat. Commun., 2021, 12 or Fernandez L, et al.; Front. Immunol., 2019, 10, followed by being induced to be differentiated into T cells by the method described above. After the cells expressing GPR56 are induced to be differentiated into T cells in advance by the above-described method, the T cells obtained by differentiation can be proliferated for 3 days to 28 days under CD3 antibody stimulation.

### <Cell Composition>

Examples of a cell composition produced by the production method of the present invention include a cell composition containing a cell expressing GPR56 that is extracted by the above-described method, and a cell composition containing a mature blood cell obtained by further inducing differentiation of the cell expressing GPR56 that is extracted by the above-described method.

A proportion of the immature blood cell expressing GPR56 that is contained in the cell composition produced by the production method of the present invention is not particularly limited, and is preferably 50% to 100% of total cells, and more preferably 80% to 100% of total cells.

The present invention relates to the cell composition containing the immature blood cell expressing GPR56, in which the proportion of the immature blood cell expressing GPR56 is 50% to 100% of the total cells. In the cell composition of the present invention, the proportion of the immature blood cell expressing GPR56 is 50% to 100% of the total cells, and preferably 80% to 100% of the total cells. When the proportion of the immature blood cell expressing GPR56 is 50% to 100% of the total cells, a cell composition in which the immature blood cells having high proliferation ability and/or high differentiation ability are concentrated with high accuracy can be obtained.

Examples of the cell contained in the cell composition of the present invention or the cells constituting the cell aggregate include, but are not particularly limited to, a blood cell, and specific examples thereof include MHSC, HSC, HPC, a T cell, and a NK cell.

The cell composition of the present invention contains a cell having high proliferation ability and/or differentiation ability, and the ability thereof can be confirmed by performing proliferation or inducing differentiation by the proliferation method or the differentiation induction method described above, and comparing the cell composition of the present invention with a cell composition in which a proportion of the immature blood cell expressing GPR56 is low.

The cell composition of the present invention can be used as a pharmaceutical cell product, a pharmaceutical composition, or a production intermediate of the cell product or the pharmaceutical composition. Examples of the pharmaceutical cell product or the pharmaceutical composition include a prophylaxis agent or a therapeutic agent for cancer, an immune disease, a blood disease or the like, or a blood preparation.

Examples of the cancer include renal cell carcinoma, Kaposi's sarcoma, chronic leukemia, prostate cancer, breast cancer, sarcoma, pancreatic cancer, rectal cancer, pharyngeal cancer, melanoma, colon cancer, bladder cancer, lymphoma, mast cell tumor, lung cancer, breast cancer, pharyngeal squamous cell Cancer, testicular cancer, Hodgkin lymphoma, gastrointestinal cancer, stomach cancer, and ovarian cancer.

Examples of the immune disease include multiple sclerosis, rheumatoid arthritis, type I diabetes, Crohn's disease, asthma, and disorders associated with allergic antibody components (for example, rheumatoid arthritis). Examples of the blood disease include aplastic anemia.

Examples of the prophylaxis agent or the therapeutic agent for a blood disease or the blood preparation include a red blood cell product, a plasma product, a platelet product, or a whole blood product, which may or may not further contain human-derived blood.

When using the cell composition of the present invention in a pharmaceutical cell product or a pharmaceutical composition, a dosage form is not particularly limited as long as the dosage form is suitable for a subject to be administered, and examples thereof include an injection, an internal preparation, an external preparation, a solid preparation, and a liquid preparation.

The subject to be administered is not particularly limited as long as it is an animal, and examples thereof include a monkey, a mouse, and a human. A human is preferred. When the cell composition of the present invention is administered to a human, a frequency and a dose per administration are appropriately set according to a disease, sex, body weight, surface area, and the like of the subject to be administered, and the frequency is, for example, 1 time to 10 times/day, and the dose per administration is, for example, 10000 cells to 100000000 cells/time.

The cell composition of the present invention may contain, in addition to the cell, for example, a medium component, a buffer agent, an excipient, or the like, which can be generally added for medically administering the cell product to a patient or stably maintaining the cell composition as a production intermediate in cell production.

Specifically, the cell composition of the present invention may contain, for example, a medium component for cell culture containing heparin or cytokines, a component secreted from a cell, a component constituting a cell such as a nucleic acid, a protein, or an amino acid, or a component constituting an extracellular matrix. The cell composition of the present invention may contain a component for medically administering a cell product to a patient, for example, a component such as an immunosuppressive agent.

The method for producing a cell composition of the present invention is not particularly limited as long as it is a method for producing a cell composition having a high proportion of immature blood cells expressing GPR56. For example, the cell composition can be produced using a production method including the above-described step of extracting cells expressing GPR56 from a cell aggregate containing immature blood cells.

That is, the cell composition of the present invention also includes a cell composition produced by a production method including the above-described step of extracting cells expressing GPR56 from a cell aggregate containing immature blood cells.

### <Kit>

One aspect of the present invention includes a kit for use in the production method of the present invention. The kit of the present invention is not particularly limited as long as it contains a compound capable of extracting cells expressing GPR56, and examples thereof include a kit containing an anti-GPR56 antibody. Particularly preferred is a kit containing an anti-CD34 antibody, an anti-CD43 antibody, and/or an anti-CD45 antibody in addition to the anti-GPR56 antibody.

The kit of the present invention can extract cells expressing GPR56 from a cell aggregate containing immature blood cells by using, for example, flow cytometry, a carrier such as beads (for example, microsphere), or affinity chromatography.

When extracting cells expressing GPR56 by flow cytometry, the kit of the present invention preferably contains, for example, a fluorescently labeled anti-GPR56 antibody.

When extracting cells expressing GPR56 using a carrier such as beads, the kit of the present invention preferably includes a carrier to which a compound capable of extracting cells expressing GPR56 is bound, and particularly preferably includes a carrier to which an anti-GPR56 antibody is bound. A bond between the antibody and the carrier may be a covalent bond, a hydrogen bond, or the like, and is not particularly limited. The anti-GPR56 antibody is not necessarily directly bound to the carrier, and may be indirectly bound to the carrier.

The carrier may be beads, for example, magnetic beads, and is not limited thereto. Other simple substances such as agarose may be used instead of the beads, and the beads may be magnetic agarose beads. Various beads (for example, microspheres) are commercially available and can be used in the present invention.

When extracting cells expressing GPR56 using affinity chromatography, the kit of the present invention preferably includes a column packed with a carrier to which a compound capable of extracting cells expressing GPR56 is bound, and particularly preferably includes a column packed with a carrier to which an anti-GPR56 antibody is bound. Further, for example, a buffer for elution of cells bound to the column may be included.

### Example

Hereinafter, the present invention will be specifically described with reference to Examples, but the present invention is not limited to these Examples.

### [Example1] Study using T Cell-derived iPS Cell Line

### <Feeder-free iPS Cell Culture>

As a T cell-derived iPS cell line (hereinafter, also referred to as T-iPSCs), a TkT3V1-7 line (supplied by The University of Tokyo, described in Nishimura et al., Cell Stem Cell. 12: 774-786, 2013) was used. T-iPS cells were cultured using a StemFit AK02N medium (Ajinomoto), and then a peeling agent was added thereto to peel off T-iPSCs.

As the peeling agent, a solution prepared by adding a 0.5M-EDTA solution (pH 8) (Nacalai Tesque) to a solution obtained by diluting 1×TrypLE Select (Thermo Fisher Scientific) to 1/2 with PBS (Nacalai Tesque) and adjusting a final concentration to 0.5×TrypLE Select and 0.75 mM EDTA was used.

To an incubator coated with an iMatrix-511 silk (Laminin-511 E8) (Nippi), a StemFit AK02N medium supplemented with 10 µM Y-27632 (Nacalai Tesque) was added, the peeled cells were seeded, and culture was continued (37°C, 5% CO₂). On the next day, the medium was replaced with a StemFit AK02N medium without Y-27632 supplemented. This operation was repeated once a week to maintain and culture iPSCs in a feeder-free manner.

### <Method for Forming Embryoid Body>

A method for forming an embryoid body (EB) containing hematopoietic stem cells from iPSCs, "method for forming embryoid body (hereinafter, referred to as EB method)" is described below.

The obtained feeder-free T-iPS cells were peeled off with 0.5 × TrypLE Select and 0.75 mM EDTA, and then seeded on an ultra-low adhesion surface 6 well plate (CORNING) at 3×10⁵ cells/well. Thereafter, culture was started under a low oxygen condition (5% O₂) using a StemFit AK02N medium supplemented with 10 µM Y-27632 and 10 µM CHIR 99021 (Tocris Bioscience). A culture start date was defined as Day 0.

On the next day (Day1), a medium obtained by supplementing a StemPro34 (Thermo Fisher Scientific) medium supplemented with 1xInsurin, Transferrin, a Selenium Solution (Thermo Fisher Scientific), 1xGlutamax (Thermo Fisher Scientific), 0.2xPSG (Sigma), 0.4 mM monothioglycerol (Wako) and 50 µg/ml L(+)-ascorbic acid (PAA) (Sigma) (hereinafter, referred to as EB medium) with 50 ng/ml BMP-4 (Miltenyi Biotec), 50 ng/ml VEGF-165A (Wako), and 50 ng/ml bFGF (Wako), followed by culturing under a low oxygen condition (5% O₂).

On the further next day (Day 2), 6 µM SB431542 (Wako) was added to the medium , followed by culturing under a low oxygen condition (5% O₂). Two more days later (Day 4), the medium was replaced with an EB medium supplemented with 50 ng/ml VEGF-165A, 50 ng/ml bFGF, and 50 ng/ml SCF (Wako), followed by culturing under a low oxygen condition (5% O₂).

Two more days later (Day 6), the medium was replaced with an EB medium supplemented with 50 ng/ml VEGF-165A, 50 ng/ml bFGF, 50 ng/ml SCF, 30 ng/ml TPO (Wako), and 10 ng/ml Flt3-L (Wako).

Thereafter, the medium was replaced every 2 to 3 days, and cells were cultured under a normal condition (5% CO₂) until Day 11 or Day 12 to produce an EB containing hematopoietic stem cells.

### <Expression Analysis of Cell Surface Markers in EB>

Cell surface markers of the EB obtained by inducing differentiation of the T cell-derived iPS cell line (TkT3V1-7) into hematopoietic stem cells were analyzed by the following procedure.

Cells in the obtained EB (Day11 or Day12) were pipetted and then passed through a cell strainer. After washing with PBS containing 2% FBS (Gibco) (hereinafter, also referred to as an FACS buffer), co-staining was performed with CD235a-FITC (BD Bioscience), CD14-APC-eFluor780 (eBioscience), APC-CD43 (BD Bioscience), CD45-BV510 (BioLegend), CD34-PE-Cy7 (Abcam), and GPR56-PE (BioLegend).

After washing, the cells were resuspended in an FACS buffer containing propidium iodide (PI) (Sigma), and cell surface markers were analyzed using a BD Aria II Cell Sorter (BD Bioscience) and FlowJo v9 (BD Bioscience). The analysis results are shown in FIG. 1.

As shown in FIG. 1, it was confirmed that a part of PI(-)CD14(-)CD235a(-)CD43(+)CD45(low+) cells expressed CD34 and a part thereof expressed GPR56. The EB-derived cells expressing GPR56 are hereinafter referred to as GPR56-positive cells.

### <Evaluation of Differentiation Ability of GPR56-positive Cells to Myeloid Cells>

In order to evaluate the differentiation ability of GPR56-positive cells into myeloid cells, cells in EB (hereinafter, also referred to as EB cells) were sorted using the expression of GPR56 and CD34 as indicators, and a colony formation test was performed to evaluate the differentiation ability into myeloid cells.

From a PI(-)CD14(-)CD235a(-)CD43(+)CD45(low+) cell aggregate among EB cells obtained from the T cell-derived iPS cell line (TkT3V1-7), a CD34(-)GPR56(-) double negative (hereinafter, also referred to as DN) cell aggregate, a CD34(+)GPR56(-) single positive (hereinafter, also referred to as SP) cell aggregate, and a CD34(+)GPR56(+) double positive (hereinafter, also referred to as DP) cell aggregate were gated, separately, using a BD Aria II Cell Sorter.

MethoCult (registered trademark) H4034 Optimum (Stem Cell Technologies) was used for the colony formation test. Each of the obtained DN cell aggregate, SP cell aggregate, and DP cell aggregate was mixed with Methocult and seeded in a 3.5 cm dish at 1 ml/dish. The number of cells in each disk was about 300 cells/dish for DP, about 1000 cells/dish for SP, and about 3000 cells/dish for DN. The dishes after seeding were left standing at 37°C and 5% CO₂.

After 16 days, the number of formed colonies was measured under a microscopic mirror. A colony having a diameter larger than 1 mm and approximately 10000 or more cells is referred to as a large colony, and other colonies having a small number of cells are referred to as small colonies.

The large colony was a colony mainly containing a plurality of cell lines, such as a colony composed of granulocytes and macrophages (CFU-GM) or a colony composed of granulocytes, red blood cells, and macrophages (CFU-GEM). The small colony was a colony mainly composed of granulocytes (CFU-G) or a colony mainly composed of macrophages (CFU-M), and contained a single cell line. The results are shown in FIG. 2.

As shown in FIG. 2, significantly more large colonies were confirmed in a DP cell fraction than in an SP cell fraction, with 4.6 in DP and 1.7 in SP per 100 cells. More small colonies were confirmed in the DP cell fraction than in the SP cell fraction, with 5.5 in DP and 3.9 in SP per 100 cells. At-test was used for significance testing (*: p<0.05). From this result, it was revealed that blood cells exhibiting higher proliferation ability and/or differentiation ability in the process of differentiation into myeloid cells were concentrated in a GPR56 positive fraction.

### <Evaluation of Differentiation Ability of GPR56-positive Cells to Lymphoid Cells>

In order to evaluate the differentiation ability of GPR56-positive cells into lymphoid cells, EB cells were gated using the expression of GPR56 and CD34 as indicators, and differentiation into T cells was induced.

In the same manner as in the evaluation of differentiation ability into myeloid cells, from a PI(-)CD14(-)CD235a(-)CD43(+)CD45(low+) cell aggregate (hereinafter, also referred to as Whole) among EB cells obtained from the T cell-derived iPS cell line (TkT3V1-7), a CD34(-)GPR56(-) double negative cell aggregate (DN), a CD34(+)GPR56(-) single positive cell aggregate (SP), and a CD34(+)GPR56(+) double positive (DP) cell aggregate were gated, separately, using the BD Aria II Cell Sorter.

Each of the gated cell aggregates was induced to be differentiated into T cells as follows. Each cell aggregate was seeded at 4000 cells/well on a 48 well plate coated with DLL4 (5 µg/ml, R&D systems) and Retronectin (5 µg/ml, Takara Bio) in advance.

Culture was performed in an alpha-MEM medium (Life Technologies) supplemented with 50ng/ml SCF (Wako), 50 ng/ml IL-7 (Wako), 50 g/ml Flt3L (Wako), 100 ng/ml TPO, 30nM SDF-1α (Wako), 15µ M SB203580 (Tocris Bioscience), 55 µM 2-mercapto Ethanol (Wako), 50 µg/ml PAA, 15% FBS, and 1% PSG. A culture start date was defined as Day 0. A new plate coated with DLL4 and Retronectin was prepared every week, and cells were replated. The medium was replaced every 2 to 3 days and the culture was performed until Day 21.

On Day 21, the number of living cells was counted using trypan blue (Nacalai Tesque). A student's t-test was used for statistical analysis (*: p < 0.05, *: p < 0.01). The results are shown in FIG. 3.

As shown in FIG. 3, the number of cells increased significantly in DP compared to DN and SP.

The cells treated to induce differentiation (Day21) were co-stained with CD3-BV510 (BioLegend), CD5-PE-Cy7 (Invitrogen), CD4-BV421 (BioLegend), and APC-CD8 (BioLegend). After washing, the cells were resuspended in an FACS buffer containing propydium iodide (PI). Cell surface markers were analyzed using a BD LSRFortessa cytometer (BD Bioscience) and FlowJo v9. The results of analyzing a proportion of CD4(+)CD8(+) cells in a PI(-)CD3(+)CD5(+) cell aggregate are shown in FIG. 4.

As shown in FIG. 4, a similar tendency was observed in the DP cell aggregate, the SP cell aggregate, and the PI(-)CD14(-)CD235a(-)CD43(+)CD45(low+) cell aggregate (referred to as "Whole"). Specifically, CD3(+) CD5(+) cells accounted for about 80% in all cell fractions of DP, SP, and Whole, and CD4(+)CD8(+) cells accounted for about 80% in the CD3(+)CD5(+) cell fraction.

From these results, it was revealed that blood cells exhibiting higher proliferation ability and/or differentiation ability in the process of differentiation into T cells were concentrated in a GPR56 positive fraction. It was revealed that, in the production of myeloid cells and the production of T cells using the T cell-derived iPS cell line, target cells can be stably produced in a large amount by extracting GPR56-positive cells.

### [Example 2] Study using iPS cell line derived from blood cells other than T cells

The same study as in Example 1 was performed using three iPS cell lines (hereinafter, referred to as Non-T-iPSCs) established from blood cells other than T cells. As Non-T-iPSC, a peripheral blood-derived iPS cell line (Ff-I01s04, supplied from Kyoto University iPS cell research office) and a cord blood-derived iPS cell line (Ff-WJs513 and Ff-WJs524, supplied from Kyoto University iPS cell research office) were used.

### <Expression Analysis of EB Cell Surface Markers>

EB cells induced from Non-T-iPSCs were co-stained in the same manner as in Example 1, and cell surface markers were analyzed in the same manner as in Example 1. The analysis results are shown in FIG. 5.

As shown in FIG. 5, it was confirmed that, in all three non-T-iPSC lines, a part of PI(-)CD14(-)CD235a(-)CD43(+)CD45(low+) cells expressed CD34 and a part thereof expressed GPR56 as in T-iPSCs.

From these results, it was revealed that, regardless of the derivation of the iPS cell line, a cell expressing GPR56 was present in the EB cells obtained by inducing differentiation of iPS cells into hematopoietic stem cells.

### <Evaluation of Differentiation Ability of GPR56-positive Cells to Myeloid Cells>

EB cells obtained from the three non-T-iPSC lines were used to evaluate the differentiation ability into myeloid cells in the same manner as in Example 1. The results are shown in FIG. 6.

As shown in FIG. 6, in all three lines, more large colonies were formed in a DP cell fraction than in an SP cell fraction, and the number of large colonies was confirmed in the DP cell fraction of Ff-I01s04 to be 9.25 times that of the SP cell fraction, in the DP cell fraction of Ff-WJs513 to be 7.68 times that of the SP cell fraction, and in the DP cell fraction of Ff-WJs524 to be 3.62 times that of the SP cell fraction. In all three lines, a significantly more small colonies were formed in the DP cell fraction than in the SP cell fraction, and the number of small colonies was confirmed in the DP cell fraction of Ff-I01s04 to be 2.775 times that of the SP cell fraction, in the DP cell fraction of Ff-WJs513 to be 1.69 times that of the SP cell fraction, and in the DP cell fraction of Ff-WJs524 to be 2.18 times that of the SP cell fraction.

From these results, it was revealed that even in the case of the EB cells derived from Non-T-iPSCs, blood cells exhibiting higher proliferation ability and/or differentiation ability in the process of differentiation into myeloid cells were concentrated in a GPR56-positive fraction.

### <Evaluation of Differentiation Ability of GPR56-positive Cells to Lymphoid Cells>

The differentiation ability of GPR56-positive cells in the EB cell aggregate obtained from Non-T-iPSCs (Ff-I01s04) into NK cells was evaluated. In the same manner as in Example 1, a DN cell aggregate, a SP cell aggregate, and a DP cell aggregate were gated from the EB cell aggregate obtained from Non-T-iPSCs, separately.

The same method as for inducing differentiation into T cells described in Example 1 was used to induce differentiation into NK cells. On Day 21, the number of living cells was counted using trypan blue. The analysis results for two independent experiments are shown in FIG. 7.

As shown in FIG. 7, all analysis results showed that the number of cells in the DP cell aggregate increased more than the DN cell aggregate and the SP cell aggregate.

The cells treated to induce differentiation (Day21) were co-stained with CD3-BV510 (Biolegend), CD7-FITC (BioLegend), CD56-APC-Cy7 (BioLegend), and APC-CD8 (BioLegend).

After washing, the cells were resuspended in an FACS buffer containing PI, and cell surface markers were analyzed using a BD LSRFortessa cytometer and FlowJo9. The results of analyzing a proportion of CD56(+)CD161(+) cells in the PI(-)CD3(-)CD7(+) cell aggregate are shown in FIG. 8.

As shown in FIG. 8, NK cells were detected in a part of the cell aggregate from the cell fractions of all the DP cell aggregate, the SP cell aggregate, and the PI(-)CD14(-)CD235a(-)CD43(+)CD45(low+) cell aggregate (referred to as "Whole"), and CD3(-)CD7(+) cells accounted for approximately 80% in all the cell fractions.

Further, CD56(+)CD161(+) cells were observed in a part of the cell aggregate of the CD3(-)CD7(+) cell fraction, and a proportion thereof was 20.2% in DP, 6.18% in SP, and 16% in whole. The abundance of NK cells in the DP cell aggregate was approximately three times that of the SP cell aggregate.

From these results, it was revealed that blood cells exhibiting higher proliferation ability and/or differentiation ability in the process of differentiation into NK cells were concentrated in a GPR56 positive fraction. From the above, it was revealed that even in the production of myeloid cells and the production of NK cells using the Non-T-iPSC line, target cells can be stably produced in a large amount by extracting GPR56-positive cells.

Although the present invention has been described in detail with reference to specific embodiments, it will be apparent to those skilled in the art that various changes and modifications can be made without departing from the spirit and scope of the invention. The present application is based on a Japanese Patent Application No. 2021-094716 filed on June 4, 2021, the entire contents of which are incorporated herein by reference. All references cited herein are incorporated in their entirety.

## Claims

1. A method for producing a cell composition, the method comprising: a step of extracting cells expressing a G protein-coupled receptor 56 (hereinafter, referred to as GPR56) from a cell aggregate containing immature blood cells.

2. The production method according to claim 1, wherein the cell expressing GPR56 is a human cell.

3. The production method according to claim 1, wherein the cell expressing GPR56 is a cell expressing CD34 along with GPR56.

4. The production method according to claim 1, wherein the cell expressing GPR56 expresses at least one of CD43 and CD45 along with GPR56.

5. The production method according to claim 1, wherein the cell aggregate is a cell aggregate derived from pluripotent stem cells.

6. The production method according to claim 5, wherein the cell aggregate is a cell aggregate obtained by inducing differentiation of the pluripotent stem cells into mesoderm and then culturing the mesoderm in a medium containing a stem cell factor (SCF), a thrombopoietin recombinant protein (TPO), and an Fms-related tyrosinase kinase 3 ligand (Flt3 -L).

7. The production method according to claim 5, wherein the cell aggregate derived from the pluripotent stem cells is a cell aggregate obtained by inducing differentiation of the pluripotent stem cells into hematopoietic stem cells or hematopoietic progenitor cells.

8. The production method according to claim 1, wherein the cell aggregate is a cell aggregate containing at least one of hematopoietic stem cells and hematopoietic progenitor cells.

9. The production method according to claim 1, wherein the cell aggregate is a cell aggregate containing cells expressing at least one selected from CD34, CD43, and CD45.

10. The production method according to claim 1, further comprising: a step of inducing differentiation of the cells expressing GPR56 into mature blood cells.

11. The production method according to claim 10, wherein the mature blood cell is a lymphoid cell.

12. The production method according to claim 11, wherein the lymphoid cell is a T cell or a natural killer cell.

13. The production method according to claim 11, wherein the lymphoid cell is a cell expressing a chimeric antigen receptor.

14. The production method according to claim 10, wherein the mature blood cell is a myeloid cell.

15. The production method according to claim 10, further comprising: a step of culturing and proliferating cells before or after inducing differentiation into the mature blood cells.

16. A cell composition comprising: an immature blood cell expressing GPR56, wherein a proportion of the immature blood cell expressing GPR56 is 50% to 100% of total cells.

17. The cell composition according to claim 16, wherein the immature blood cell expressing GPR56 is a human cell.

18. The cell composition according to claim 16, wherein the immature blood cell expressing GPR56 is a hematopoietic stem cell or a hematopoietic progenitor cell.

19. The cell composition according to claim 16, wherein the immature blood cell expressing GPR56 is a cell expressing CD34 along with GPR56.

20. The cell composition according to claim 16, wherein the immature blood cell expressing GPR56 is a cell expressing at least one of CD43 and CD45 along with GPR56.

21. The cell composition according to claim 16, wherein the immature blood cell expressing GPR56 is a cell derived from a pluripotent stem cell.

22. The cell composition according to claim 21, wherein the immature blood cell expressing GPR56 is a cell obtained by inducing differentiation of the pluripotent stem cell into mesoderm and then culturing the mesoderm in a medium containing SCF, TPO, and Flt3-L.

23. The cell composition according to claim 21, wherein the immature blood cell expressing GPR56 is a cell obtained by inducing differentiation of the pluripotent stem cell into a hematopoietic stem cell or a hematopoietic progenitor cell.

24. A pharmaceutical composition comprising: a cell composition produced by the production method according to any one of claims 1 to 15.

25. The pharmaceutical composition according to claim 24, wherein the pharmaceutical composition is a therapeutic agent or blood preparation for at least one selected from cancer, an immune disease, and a blood disease.

26. A pharmaceutical composition comprising: the cell composition according to any one of claims 16 to 23.

27. The pharmaceutical composition according to claim 26, wherein the pharmaceutical composition is a therapeutic agent or blood preparation for at least one selected from cancer, an immune disease, and a blood disease.
